Europäisches Patentamt

European Patent Office

Office européen des brevets

(11) Publication number: **0 336 647 B1**

# EUROPEAN PATENT SPECIFICATION

(45) Date of publication of patent specification: **13.07.94**  (51) Int. Cl.5: **G01N  33/88**, G01N 33/543

(21) Application number: **89303163.3**

(22) Date of filing: **30.03.89**

(54) **Determination of arachidonic acid derivatives and kits therefore.**

(30) Priority: **31.03.88 JP 79006/88**
**25.08.88 JP 209389/88**

(43) Date of publication of application:
**11.10.89 Bulletin  89/41**

(45) Publication of the grant of the patent:
**13.07.94 Bulletin  94/28**

(84) Designated Contracting States:
**AT BE CH DE ES FR GB GR IT LI LU NL SE**

(56) References cited:
**EP-A- 0 166 583**

**PROSTAGLANDINS, vol. 33, 1987, Butterworth, Stoneham,MA (US); H.L. HUBBARD et al., pp. 149-160&NUM;**

**CHEMICAL ABSTRACTS, vol. 108, no. 13, 28 March 1988, Columbus, OH (US); M. SAWADA et al., p. 325, no. 109131t&NUM;**

**ADVANCES IN PROSTAGLANDIN, THROMBOXANE, AND LEUKOTRIENE RESEARCH, vol. 11, 1983, B. Samuelsson, R. Paoletti & P. Ramwell, Raven Press, New York, NY (US); S. YAMAMOTO et al., pp. 181-184&NUM;**

(73) Proprietor: **Sankyo Company Limited**
**5-1 Nihonbashi Honcho 3-chome**
**Chuo-ku**
**Tokyo(JP)**

Proprietor: **ONO PHARMACEUTICAL CO., LTD.**
**1-5, Doshomachi 2-chome**
**Chuo-ku Osaka 541(JP)**

(72) Inventor: **Nakamura, Kanichi**
**Sankyo Co. Limited**
**No. 2-58 1-chome**
**Hiromachi**
**Shinagawa-ku Tokyo 140(JP)**
Inventor: **Takahagi, Hidekuni**
**Sankyo Co. Limited**
**No. 2-58 1-chome**
**Hiromachi**
**Shinagawa-ku Tokyo 140(JP)**
Inventor: **Nakagawa, Akihiko**
**Sankyo Co. Limited**
**No. 2-58 1-chome**
**Hiromachi**
**Shinagawa-ku Tokyo 140(JP)**

Inventor: **Takasaki, Wataru**
**Sankyo Co. Limited**
**No. 2-58 1-chome**
**Hiromachi**
**Shinagawa-ku Tokyo 140(JP)**

(74) Representative: **Gibson, Christian John Robert**
**et al**
**MARKS & CLERK,**
**57/60 Lincoln's Inn Fields**
**London WC2A 3LS (GB)**

EP 0 336 647 B1

**Description**

BACKGROUND OF THE INVENTION

This invention relates to methods for determination of arachidonic acid derivatives, and in particular to methods for determining arachidonic acid derivatives selected from prostaglandins, thromboxanes and leukotrienes. The present invention also relates to kits for use in such determinative methods.

Prostaglandins, thromboxanes and leukotrienes (generally referred to herein as arachidonic acid derivatives) have potent physiological activity even in small amounts and have been implicated in the etiology of various diseases. Many analytical methods for quantitatively determining their presence in body fluids have been reported. The analytical methods can be classified broadly into chromatographic methods and immunoassays.

Chromatographic methods adopted for analysis include thin layer chromatography ("TLC"), high performance liquid chromatography ("HPLC"), gas chromatography ("GC"), and gas chromatography-mass spectrometry ("GC-MS"). In general, results of high reliability can be obtained to some extent, even for structurally similar compounds. The GC-MS method is the most sensitive method, but it remains difficult to quantitate a level of 10 pg/ml in plasma.

For clinical diagnostic purposes, a clean-up pretreatment is necessary, requiring cumbersome and complicated techniques such as extraction from a body fluid sample and conversion to a derivative suited for analytical measurement. For this reason, a large amount of sample is required. Furthermore, the number of samples which can be measured at one time is limited, the equipment necessary for analysis is expensive, and such methods cannot be said to be of wide applicability.

Immunoassays include radioimmunoassay ("RIA") and enzyme immunoassay ("EIA"). Respective examples of radioimmunoassay and enzyme immunoassay for prostaglandins are to be found, for instance, in the minireview Radioassay of Prostaglandins (Prostaglandins (1978) 15, 3) and in EP166583. With such methods, it is possible to detect a compound at a plasma level of around 1 pg/ml. However, an immunoassay is not a separative technique, and there is the risk of cross-reactivity. When substances are present which interfere with the antigen-antibody reaction, considerable errors can arise.

Accordingly, it is known to employ a chromatographic purification and clean-up pretreatment before carrying out an immunoassay. Specifically, Powell (Prostaglandins (1980) 20, 947) report the use of a column packed with a silica gel modified with octadecyl groups. This clean-up method has been adopted by other workers, see, for example, A Solid-Phase Enzyme Immunoassay of Thromboxane $B_2$ (J Biochem (1985) 98, 1069); Enzyme Immunoassay of Thromoboxane $B_2$ (Biochim Biophys Acta (1983) 750, 322); Development of Enzyme Immunoassay For Serum 13,14-Dihydro-15-ketoprostaglandin (Biochim Biophys Acta (1986) 879, 322); and Enzyme Immunoassay of 6-Ketoprostaglandin $F_{1\alpha}$ In A Solid Phase (Biochim Biophys Acta (1985) 836, 335).

There have also been developed a large number of methods in which solvent extraction, thin layer chromatography, high performance liquid chromatography, and affinity chromatography are used alone or in combination, in order to provide a cleaned sample for immunoassay. In this respect, reference is made, for instance, to Enzyme Immunoassay Of Prostaglandin $F_{2\alpha}$ (Biochim Biophys Acta (1981) 663, 661); Extraction Of Thromboxane $B_2$ From Urine Using An Immobilized Antibody Column For Subsequent Analysis By Gas Chromatography-Mass Spectrometry (Prostaglandins (1987) 33, 149); Antibody-Mediated Extraction/Negative-Ion Chemical Ionization Mass Spectrometric Measurement Of Thromboxane $B_2$ And 2,3-Dinor-Thromboxane $B_2$ In Human And Rat Urine (Anal Biochem (1987) 163, 255); and Simultaneous Dermination of Thromboxane $B_2$ And 6-Ketoprostaglandin $F_{1\alpha}$ In Plasma By Gas Chromatography/Mass Spectrometry Using Novel Clean-Up Method With Immobilized Antibody (Abstracts 23rd Int Symp Adv Chromatog (1986) L20, 39).

However, among these combined clean-up/immunoassay methods, the simple methods are generally poor in selectivity and cannot overcome the existing problems, such as cross-reactivity, while the more sophisticated methods intended to improve selectivity are generally cumbersome and also lower in recovery and reproducibility.

Furthermore, with radioimmunoassays, there is the disadvantage that radioisotopes are used, and have to be handled by qualified personnel in a specific containment facilities. There is the drawback that a stable measuring system can only be maintained with difficulty, because the radioisotopes decay with time.

Another problem encountered with prostaglandins is illustrated by prostaglandin $E_2$ ("$PGE_2$"), which is a primary prostaglandin and is an important compound having a wide range of physiological activities. Determination of its level in patients might become a significant diagnostic guide for treatment. However, due to poor stability, prostaglandin $E_2$ undergoes decomposition during many clean-up procedures, and

3

accurate quantitation is often difficult.

It is known to avoid some problems by assaying for mimics or metabolites of the prostaglandin or other arachidonic acid derivative of interest. However, even this approach is not without difficulties. For example, thromboxane $A_2$ is of interest for its platelet aggregation promoting action and other activities, and can be indirectly assayed by the use as mimic of thromboxane $B_2$ (hereinafter abbreviated as "TXB$_2$"), which is generally understood to be a stable metabolite. However, it is known that the concentration of TXB$_2$ will vary depending on the blood sampling technique (J Chromatog (1985) $\underline{338}$, 273). It is therefore necessary to assay for a metabolite, such as 11-dehydrothromboxane $B_2$ ("11-dhTXB$_2$"), whose level will not be artifactually influenced by the blood sampling technique.

Thus, as a generality, a need still remains for a combination of a clean-up purification method capable of pretreating a large number of test samples selectively with good efficiency, with an assay method capable of giving quantitative results of high reliability.

OBJECTS OF THE PRESENT INVENTION

A major object of this invention is a combined clean-up/assay method for an analyte which is an arachidonic acid derivative. One object is a determination method which is relatively simple to adopt, which can avoid the need for sophisticated equipment, and yet which is capable of giving relatively accurate results for multiple samples. Another object is a method suited for assay of problem analytes such as TXA$_2$ or PGE$_2$. A related object is the provision of kits enabling such determination method to be readily carried out by ordinary skilled technicians.

SUMMARY OF THE PRESENT INVENTION

The present invention is based on a combination of affinity chromatography for cleaning up samples, using an immobilized antibody as affinity ligand for the arachidonic acid derivative, together with an enzyme immunoassay. In particular, the inventors have found that with antibody-based affinity chromatography it is possible to remove substantially all of the intervening compounds which exhibit cross-reactivity or otherwise interfere with the antigen-antibody reaction in an immunoassay. An efficient and selective clean-up pretreatment can be performed by simple operations using simple equipment. In combination, the inventors have also found that a reasonably simple quantitation with high sensitivity of arachidonic acid derivative analytes can be effected by combining such clean-up pretreatment with an enzyme immunoassay.

PREFERRED EMBODIMENTS OF THE PRESENT INVENTION

In one aspect, the present invention provides a method for determination of an analyte in a sample, in which the analyte is an arachidonic acid derivative selected from the group consisting of prostaglandins, thromboxanes and leukotrienes.

The determination method comprises the steps of:

providing an affinity chromatography column packed with a packing material comprising an immobilized first antibody, the first antibody being immunoreactive with the analyte;

applying a sample to the column to adsorb to the packing material any analyte in the sample;

washing the column to elute non-adsorbed sample;

desorbing any adsorbed analyte from the column to give an assay fraction;

assaying for analyte in the assay fraction using an enzyme immunoassay based on reagents including an assay antibody immunoreactive with the analyte and an enzyme-labelled arachidonic acid derivative immunoreactive with the assay antibody.

The reagents for the enzyme immunoassay can further include a second antibody which is an antiglobulin immunoreactive with the assay antibody.

Examples of analytes which may be assayed by the present invention include prostaglandins, which are derivatives of prostanoic acid having the prostaglandin skeleton, and particularly the natural stable prostaglandins of the arachidonic acid cascade. Examples of such prostaglandins include specifically PGA$_1$, PGA$_2$, PGA$_3$, PGB$_1$, PGB$_2$, PGB$_3$, PGC$_1$, PGC$_2$, PGC$_3$, PGD$_1$, PGD$_2$, PGD$_3$, PGE$_1$, PGE$_2$, PGE$_3$, PGF$_{1\alpha}$, PGF$_{2\alpha}$, PGF$_{3\alpha}$ PGI$_2$, or PGI$_3$, or metabolites thereof in body fluids such as PGF-MUM (main metabolite of PGF$_{2\alpha}$ in urine), PGE-MUM (main metabolite of PGE$_2$ in urine), or 6-keto-PGF$_{1\alpha}$ (metabolite of PGI$_2$).

The analytes further include thromboxanes, having the thromboxane skeleton, and particularly natural stable thromboxanes of the arachidonic acid cascade. Examples of such thromboxanes include specifically TXB$_1$, TXB$_2$, or TXB$_3$, or metabolites thereof in body fluids such as 11-dehydroTXB$_2$ (11-dhTXB$_2$,

metabolite of $TXB_2$ and useful for estimating the level of $TXA_2$).

The analytes also include leukotrienes, having the leukotriene skeleton, and particularly natural stable leukotrienes of the arachidonic acid cascade. Examples of such leukotrienes include specifically $LTA_3$, $LTA_4$, $LTA_5$, $LTB_3$, $LTB_4$, $LTB_5$, $LTC_3$, $LTC_4$, $LTC_5$, $LTD_3$, $LTD_4$, $LTD_5$, $LTE_3$, $LTE_4$, or $LTE_5$, or metabolites thereof in body fluids.

Apart from the natural compounds exemplified as analytes which can occur in body fluids, the present invention can also be used for determination of chemically synthesized derivatives obtained for instance by modification of natural compounds or metabolites thereof.

Typical analytes which may be determined by the method of the present invention, include $PGD_2$, $PGE_2$, $PGF_{2\alpha}$, $TXB_1$, $TXB_2$, $TXB_3$, $LTB_4$, $LTC_4$, $LTD_4$, and metabolites thereof, such as PGF-MUM, PGE-MUM, 11-dehydro$TXB_2$, and 2,3-dinor-$TXB_2$, and more especially $PGE_2$ or 11-dh$TXB_2$ (an index of $TXA_2$).

The effectiveness of the present invention is based on the combination of affinity chromatography with an enzyme immunoassay. The affinity chromatography will now be considered in more detail.

For the affinity chromatography, the method of this invention employs as the first antibody an antibody immunoreactive with the analyte. Such antibodies can be prepared by conventional techniques adopted for instance in some of the literature cited above. Thus, antigenic material can be prepared by coupling a suitable arachidonic acid derivative to a carrier protein. To this end, the analyte compound or a mimic therefor can be employed as the arachidonic acid derivative. A functional group of the arachidonic acid derivative, such as an existing carboxyl or hydroxy group or a newly introduced functional group, can be coupled to a functional group of the protein, such as an amino group, mercapto group or hydroxy group.

Suitable carrier proteins include albumin, globulin, thyroglobulin, hemocyanin, edestin, and the like, preferably albumin. The arachidonic acid derivative can be directly coupled with protein. For example, coupling can be effected in an appropriate solvent (for instance a phosphate buffer) by the active esterification method, the carbodiimide method, the acid anhydride method, or the like method. Preferred methods include the use of cyclohexylcarbodiimide, 1-ethyl-3-(3-dimethylaminopropyl)carbodiimide, 1,1′-oxalyldiimidazole, 2,2′-dipyridyldisulfide, N,N′-disuccinimidyl carbonate, N,N′-bis(2-oxo-3-oxazolidinyl)-phosphinic chloride, N,N′-carbonyldiimidazole, N,N′-disuccinimidyl oxalate (DSO), N,N′-diphthalimide oxalate (DPO), N,N′-bis(norbornenylsuccinimidyl) oxalate (BNO), 1,1′-bis(benzotriazolyl) oxalate (BBTO), 1,1′-bis(6-chlorobenzotriazolyl) oxalate (BCTO) or 1,1′-bis(6-trifluoromethylbenzotriazolyl) oxalate (BTBO) as the coupling agent. Alternatively, particularly when the arachidonic acid derivative can not be coupled directly to the protein, the known method using a cross-linking agent such as 1,5-difluoro-2,4-dinitrobenzene can be employed.

After the coupling reaction, the desired product can be isolated and purified by column chromatography or dialysis. The antigenic material can then be mixed with an appropriate adjuvant in suspension, and administered to an animal to sensitize the animal.

The sensitization of the animal can be performed using generally known techniques. The antigenic material is usually formed into a suspension with an adjuvant.

Known adjuvants may be employed, including Freund's complete or incomplete adjuvant, aluminum hydroxide, alum, pertussis vaccine, and mixtures thereof. Freund's incomplete adjuvant is sometimes preferred employed as a booster. The antigenic material is administered to a suitable animal (for example, rat, mouse, guinea pig, rabbit, cat, dog, sheep, goat, or other animal) at appropriate administration intervals over several weeks or months for sensitization. After sensitization, the serum of the sensitized animal is collected, followed by further processing as desired, to give the desired antibody.

In practice, and especially for body fluid samples, a large number of structural homologues will frequently exist in the test sample, in addition to the compound to be detected. Accordingly, it is most preferred to prepare an antibody having high specificity for the analyte.

A monoclonal antibody is also suitable. Such an antibody can be prepared according to known methods, for example by the procedures of immunization, cell fusion, screening, and cloning, as described for instance in Iwasaki et al. (Monoclonal Antibody, Hybridoma and ELISA, pp. 30 to 103 (Kodansha Scientific)).

In selection of the animal to be immunized for production of a monoclonal antibody, the animal species and the immune response to the antigen are important. Generally speaking, stable antibody-producing hybridomas will be frequently formed with good efficiency when the spleen cells to be used and myeloma are of the same animal species. Particularly preferred is the use of BALB/c mice. Preferred myeloma cell species include P3•X63•Ag8(X63), P3•NS-1/1•Ag4•1(NS-1), SP2/O•Ag14(SP-2) and FO.

As examples of the the cloning method, the limiting dilution method, the soft agar method, the fibrin gel method and the fluorescence activated cell sorter method can be employed, preferably the limiting dilution method or the soft agar method.

For affinity chromatography, an affinity chromatography column is packed with the packing material comprising the immobilized first antibody. Known immobilization techniques and materials can be employed. Examples of immobilization methods include the physical adsorption method, the ion bonding method, the covalent bonding method, the support crosslinking method, the support-less crosslinking method, and the inclusion method. In general, the first antibody is usually immobilized on a support to give the packing material, though when the first antibody is immobilized by the support-less crosslinking method, the packing material typically comprises only the immobilized first antibody.

The support may be one generally used, and the choice is not particularly limited. Selection of the support depends on the properties of the antibody to be immobilized, but it is also necessary to consider the size of particles, the surface area in the three-dimensional network structure, the ratio of hydrophilic sites to hydrophobic sites, chemical composition, strength to pressure, etc, of the support. Typical examples of the support include polysaccharide derivatives such as cellulose, dextran, or agarose; synthetic polymers such as polyacrylamide gel, or polystyrene resin; and inorganic materials such as porous glass, or metal oxide.

With the physical adsorption method, where the antibody is immobilized by physical adsorption onto a water-insoluble support, examples of particularly preferred supports include inorganic substances such as activated charcoal, porous glass, acidic white clay, bleached clay, kaolinite, alumina, silica gel, bentonite, hydroxyapatite, calcium phosphate, metal oxide, or ceramic; a natural polymer such as starch or gluten; or a porous synthetic resin. Adsorption hydrophobically onto a support having hydrophobic groups such as butyl- or hexyl-Sephadex is also possible (Sephadex is a Trade Mark).

With the ion bonding method, where the antibody is immobilized by binding ionically to a water-insoluble support having ion exchange groups, particularly preferred examples of the support include polysaccharides having ion exchange groups such as DEAE-Sephadex or synthetic polymer derivatives such as ion exchange resins.

With the covalent bonding method, where the antibody is immobilized by binding covalently to a water-insoluble support, examples of particularly preferred supports include those having amino, carboxyl, sulfhydryl, hydroxy, imidazole or phenol groups which are functional groups reactive for instance with diazonium salts, acid azides, isocyanates, or active type alkyl halides.

With the support crosslinking method, where the antibody is immobilized to the support by covalent binding with the use of a crosslinking reagent such as glutaraldehyde, examples of particularly preferred supports include water-insoluble supports having amino groups, such as AE-cellulose, DEAE-cellulose, partially deacylated chitin, or aminoalkylated porous glass.

With the support-less crosslinking method, where immobilization is effected by crosslinking antibodies with a reagent having two or more functional groups, no support is particularly required. Examples of preferred crosslinking reagents include glutaraldehyde (forming a Shiff's base), an isocyanic acid derivative (forming a peptide), N,N'-ethylenebismaleimide, bisdiazobenzidine (for diazo coupling), or N,N'-polymethylenebisiodoacetamide (alkylating agent). The antibody which participates in the crosslinking reaction needs a suitable functional group at the N-end, such as an amino group, phenol group or sulfhydryl group or imidazole group.

With the inclusion method, the method may be classified into the lattice type in which antibodies are incorporated into fine lattices of polymeric gels, and the microcapsule type in which the antibodies are coated with semipermeable polymeric films. Examples of preferred supports in the case of the lattice type include polymeric compounds, for example, synthetic polymeric substances such as polyacrylamide gel, polyvinyl alcohol, or photocurable resin; and natural polymeric substances such as starch, konjak powder, gelatin, alginic acid, or carageenan. In the case of the microcapsule type, various techniques are possible. When the interfacial polymerization method is used, namely the method in which the antibody is coated by utilizing the principle of polymerizing a hydrophilic monomer and a hydrophobic monomer at the interface therebetween, a nylon film based on hexamethylenediamine and sebacoyl chloride can be employed. When the drying-in-liquid method is used, namely the method in which an antibody solution is dispersed in a polymeric compound solution dissolved in an organic solvent to form an emulsion and then transferred into an aqueous solution followed by drying, thereby coating the antibody, examples of preferred supports include polymeric substances such as ethyl cellulose or polystyrene. When the phase separation method is used, namely the method in which a polymeric compound is dissolved in an organic solvent immiscible with water, an antibody is dispersed in the solution to prepare an emulsion, then a non-solvent which causes phase separation is gradually added under stirring, whereby a concentrated solution of the polymeric compound encloses the antibody droplets therearound, and subsequently the polymeric compound is precipitated to form a film which covers the antibody, is used, the above-mentioned polymeric compounds can be employed.

As examples of commercially available supports there may be specifically illustrated supports such as those known by the Trade Marks AF-Epoxytoyopal 650M, AF-Aminotoyopal 650M, AF-Formyltoyopal 650M, AF-Carboxytoyopal 650M (all produced by Toyo Soda Mfg. Co., Ltd.), Reacti-Gel (6X, 25DF, HW-65F and GF-2000), CPG/CDI-Activated Glycophase and Hydrazide Beads, Alkylamine Beads, Sanger Reagent Beads (all produced by Pierce Co.), Mini Leak (produced by Kem-En-Tec Co.), Eupergit C (30N, 250L and 1Z) (all produced by Rohm Pharma Co.), Actigel (A,A-Superflow, B, H and T), Aminogel (A, B), Carboxygel, Thiogel and Diazo-gel (all produced by Sterogene Co.), Affigel (10, 15, 102, 202, 401, 501, 601 and 731), Affiprep (10), Aminoethyl Biogel (P-2 and P-100) and CM Biogel A (all produced by Biorad Co.), CNBr-activated Sepharose 4B, Tresyl-activated Sepharose 4B, CH-Sepharose 4B, AH-Sepharose 4B, Activated CH-Sepharose 4B, Epoxy-activated Sepharose 6B, Activated Thiol-Sepharose 4B, CNBr-activated Sepharose 6MB (all produced by Pharmacia Co.). The present invention is not limited to these examples.

In practice the experimental method can be conducted following the methods described by Fukui et al. (Enzyme Engineering, pp. 164 to 243, 1981, Tokyo Kagaku Dojin).

The affinity chromatography column, which can be a disposable or a reusable column, is packed with the packing material. Conventional columns for operation at normal or higher pressure can be used, such as columns of glass or plastics material, preferably those made of a plastics material and of about 5 ml volume. Illustrative examples of commercially available products include Sepacolmini PP (produced by Seikagaku Kogyo Co.), Econocolumn (produced by Biorad Co.), Disposal Polystyrene Column (produced by Pierce Co.), Microcolumn (produced by Whale Co.), etc. A column comprising a packed Pasteur pipet with a stopper of glass wool can also be used.

The affinity chromatography can be practiced according to the following steps:

(1) The immobilized antibody is packed in the column.

(2) A test sample is brought into contact with the immobilized antibody so that analyte in the test sample may be adsorbed by the immobilized antibody.

(3) Substances which may interfere with the subsequent assay are washed away from the column.

(4) An eluent for eluting the analyte from the immobilized antibody is applied to the column. This eluent is typically an aqueous solution containing 10% or more (which may be 100%) of a polar organic solvent as exemplified by methyl alcohol, acetonitrile, acetone, or the like.

By means of the affinity chromatography, a cleaned sample is obtained which is particularly suited for enzyme immunoassay, in accordance with the present invention.

The enzyme immunoassay can be performed using known techniques, and is generally performed as a heterogenous enzyme immunoassay, involving separating antibody-bound analyte and/or antibody-bound enzyme-labelled compound from free analyte and/or free enzyme-labelled compound. For example, the separation of the bound form (B form) from the unbound, free form (F form) can be practiced by the double antibody method or the solid phase antibody method, which are generally known methods (see Eiji Ishikawa, Tadashi Kawai, Kiyoshi Miyai, ed., Enzyme Immunoassay, 3rd edition, Igaku Shoin, 1987). The enzyme activity can then be determined, permitting a quantitative estimation of the analyte. To this end, the assay ordinarily employs a calibration based on standard solutions of known analyte concentration.

The enzyme immunoassay is effected using the assay antibody immunoreactive with the analyte, and the enzyme-labelled arachidonic acid derivative immunoreactive with the assay antibody, and usually also employs a second antibody which is an antiglobulin immunoreactive with the assay antibody. The use of such a second antibody allows the use of the double antibody method for enzyme immunoassay, which is preferred.

The assay antibody can be prepared by the techniques already described for the first antibody. The enzyme-labelled compound is prepared by labelling a suitable arachidonic acid derivative with an enzyme. The arachidonic acid derivative may be the analyte measured, or a mimic (such as 9-deoxy-9-methylene-$PGF_{2\alpha}$ as a mimic for $PGE_2$). The enzyme may be any of the enzymes generally used in enzyme immunoassay, including maleate dehydrogenase, glucose-6-phosphoric acid dehydrogenase, glucose oxidase, peroxidase, acetylcholine esterase, alkali phosphatase, glucoamylase, lysozyme, $\beta$-D-galactosidase, etc., preferably peroxidase, alkali phosphatase or $\beta$-D-galactosidase. The labelling may be performed in a manner similar to the reaction for coupling an arachidonic acid derivative to the carrier protein, as described above in respect of the methods for preparation of the first antibody. Preferably, the active esterification method, the mixed acid anhydride method using isobutyl chloroformate, or the method using 4-(N-maleimidomethyl)cyclohexane-1-carboxylic acid N-hydroxysuccinimide ester as the crosslinking agent is adopted.

In this case, the specificity of the enzyme-labelled compound may be enhanced. If necessary, the assay sensitivity can be enhanced by varying the amount of arachidonic acid derivative bound to the enzyme.

EP 0 336 647 B1

When used, the second antibody employed in the enzyme immunoassay can be specifically prepared by sensitization of a different species of animal through administering a serum or $\gamma$-globulin of the same species of animal used during preparation of the first antibody. However an antibody from a commercially available serum of a different species of animal may be utilized.

The second antibody can be used without a support (the double antibody liquid phase method) or as a solid phase support-bound product (the double antibody solid phase method).

Binding of the second antibody to the solid phase can be achieved using physical adsorption or chemical binding, as described above. The material for the solid phase may be any of those generally used for enzyme immunoassays, including polysaccharides such as agarose, dextran and cellulose, synthetic resins such as polystyrene, glass or polyacrylamide, and so on. The shape may be any form, provided that it can be easily separated, but preferred shapes include small spheres, small test tubes, tubes, fibrous shapes and microplates. For preference, polystyrene balls or glass beads may be employed (as to details, see Ichiro Chihata, ed., Immobilized Enzyme, Kodansha, 1975).

With the double antibody method, the assay method can be practiced by:

(1) mixing a first antibody, an enzyme-labelled antigen and a cleaned-up test sample to carry out competitively the antigen-antibody reaction (called the first reaction), adding supported or unsupported second antibody (respectively for the solid phase or liquid phase method) to effect binding (called the second reaction), and then assaying the enzyme activity of the bound enzyme-labelled antigen, or

(2) subjecting initially a first antibody and a cleaned-up test sample to the antigen-antibody reaction (called the first reaction), adding supported or unsupported second antibody (respectively for the solid phase or liquid phase method) to effect binding (called the second reaction), then adding an enzyme-labelled antigen to effect binding (called the third reaction), and assaying the enzyme activity of the bound enzyme-labelled antigen.

Either method is preferable, and it is necessary to choose one of the methods depending on the kind of the substance to be assayed.

The first reaction in the method (1) and the first reaction and the second reaction in the method (2) are suitably carried out at 4 to 50°C for 1 to 4 hours. With the reaction temperature and time within this range, a stable assay is possible with the assay sensitivity not influenced thereby. The second reaction in the method (1) and the third reaction in the method (2) may typically be carried out at 4°C overnight.

With the solid phase antibody method, the assay method can be practiced by:

(3) adding an enzyme-labelled antigen and a cleaned-up test sample to a supported assay antibody to carry out competitively the antigen-antibody reaction (called the first reaction), and then assaying the remaining enzyme activity of the enzyme-labelled antigen after removing the unbound fraction.

The first reaction in the method (3) may typically be carried out 4 to 50°C for 1 to 24 hours."

Determination of the enzyme activity of the enzyme-labelled antigen may be practiced according to known methods (see Eiji Ishikawa, Tadashi Kawai, Kiyoshi Miyai, ed., Enzyme Immunoassay, 3rd edition, p.21, Igaku Shoin, 1987).

In a further aspect, the present invention provides kits for determination of an analyte in a sample, where the analyte is an arachidonic acid derivative which is selected from prostaglandins, thromboxanes and leukotrienes.

The kit includes:

an affinity chromatography column and a packing material comprising an immobilized first antibody, said first antibody being immunoreactive with said analyte;

an assay antibody immunoreactive with the analyte;

an enzyme-labelled arachidonic acid derivative immunoreactive with the assay antibody; and

optionally, a supported or unsupported second antibody which is an antiglobulin immunoreactive with the assay antibody.

Standard samples of analytes, reagents for assay of activity of the labelled enzyme, buffering agents, enzyme reaction stopping solution, and/or other materials can optionally be incorporated in the kits for quantitation of the analyte and can be prepared according to known methods.

Various advantages stem from the combination of affinity chromatography and enzmye immunoassay provided by the present invention. For example, the clean-up method does not require special skills such as are needed with solvent extraction, high performance liquid chromatography, or gas chromatography, and is easy to operate and automate. The immobilized antibody column can be reutilized by washing with a polar organic solvent. The enzyme immunoassay can itself be automated by use of microtiter plates, and a large number of assays are possible at one time, unlike the gas chromatography-mass spectrometry method. No handling by qualified personnel in a specific containment facility, as in the radioimmunoassay method, is required, and the kit reagents are stable over a long term.

8

EXAMPLES OF THE INVENTION

Embodiments of the present invention are described in more detail in the following non-limiting Examples, with reference to the accompanying drawings.

DESCRIPTION OF THE DRAWINGS

Figure 1 shows the recovery of 11-dhTXB$_2$ in Example 1(4).

Figure 2 is a standard calibration curve for 11-dhTXB$_2$, obtained in Example 1(6).

Figure 3 shows the recovery of 11-dhTXB$_2$ in Example 2(6).

Figure 4 shows the recovery of PGE$_2$ in Example 3(4).

Figure 5 is a standard calibration curve for PGE$_2$, obtained in Example 3(6).

<u>Example 1</u>

Assay of 11-dehydrothromboxane B$_2$ (11-dhTXB$_2$) in plasma

(1) Preparation of antigen

To a solution of 15 mg of the lactone form of 11-dhTXB$_2$ in 0.45 ml of acetonitrile were added 11.4 mg of N,N′-disuccinimidyl carbonate and 6.2 $\mu$l of triethylamine, and the mixture was mixed at room temperature for 10 minutes. 5 ml of ethyl acetate was added, and the mixture was washed with aqueous saturated sodium hydrogen carbonate, then with aqueous sodium chloride, and dried over anhydrous sodium sulfate. 7.75 mg of the N-succinimidyl ester of 11-dhTXB$_2$ obtained by evaporation of the solvent was dissolved in 0.78 ml of pyridine, and the resulting solution was mixed with a solution of 37 mg of bovine serum albumin (hereinafter referred to as "BSA") dissolved in 0.78 ml of 0.05M phosphate buffer (pH 7.3, hereinafter referred to as "PB"). The mixture was then stirred at 4°C for 24 hours.

The reaction mixture was made up to a total volume of 3 ml by the addition of aqueous 50% dimethylformamide, dialyzed against aqueous 50% dimethylformamide for 24 hours, and then against physiological saline for 48 hours, at 4°C in both cases. To 36 mg of the resultant prepared antigen was added 18 ml of physiological saline, and the saline antigen solution was stored at -20°C.

The mol ratio of 11-dhTXB$_2$:BSA in the antigen was found by adding $^3$H-11-dhTXB$_2$ to the reaction system, and determined to be 12:1.

(2) Preparation of first antibody

Injections for immunization of a white male domestic rabbit were prepared using 0.5 ml of the aqueous physiological saline solution of the prepared antigen mixed with 0.5 ml of Freund's complete adjuvant. Immunization of the rabbit was commenced by subcutaneous injection at the back and the paw. Immunization was then continued by subcutaneous injection at the back every other week for 7 times in total. After 10 days from the final injection, 1 ml of an aqueous solution containing 1% of indomethacin and 0.3% of sodium hydrogen carbonate was intravenously injected, and then blood was collected from the heart of the rabbit. After the blood had been left to stand at room temperature for 5 hours, the serum providing the first antibody was collected by centrifugation.

The cross-reactivity (%) of the antibody in the serum was determined as shown in the following Table 1:

EP 0 336 647 B1

Table 1

| 11-dhTXB$_2$ (acid form) | 100 |
|---|---|
| TXB$_2$ | 0.01 |
| PGD$_2$ | 0.05 |
| PGE$_1$ | <0.01 |
| PGE$_2$ | <0.01 |
| PGF$_{1\alpha}$ | <0.01 |
| PGF$_{2\alpha}$ | <0.01 |
| 6-keto-PGF$_{1\alpha}$ | <0.01 |
| 6-keto-PGF$_{2\alpha}$ | <0.01 |

(3) Preparation of immobilized antibody column

The ammonium sulfate fraction was obtained from 20 ml of the serum containing the first antibody, and the IgG fraction was prepared by DE-52 column chromatography according to a known method (Biological Experimental Course (5), Continued, Immunobiochemical Study Method, pp. 11 to 24, edited by Biochemical Society of Japan, Tokyo Kagaku Dojin (1986)). 200 mg of the IgG fraction (by weight of protein) was sealed into a dialyzing membrane, and concentrated using polyethylene glycol 2000 by dialysis against 0.1M carbonate buffer (pH 9.0) at 4°C for 24 hours. Immobilization was performed according to a known method (supra, pp. 29 to 31), which will now be described. That is, 70 ml of water was added to 40 g of Sepharose 4B and, with ice-cooling, 4.5 g of finely divided cyanogen bromide was added while maintaining the pH at 11.0 to 11.5, followed by mixing. When the pH no longer needed to be maintained, the Sepharose 4B material was washed with a 20-fold excess by volume of 0.1M carbonate buffer (pH 9.0) to obtain Sepharose 4B activated with cyanogen bromide. To this was added the dialyzed IgG fraction, and the mixture was stirred at 4°C for 24 hours. The mixture was washed with 0.1M Tris-hydrochloride buffer (pH 8.0), which was then replaced with phosphate buffered saline (hereinafter referred to as "PBS") (pH 7.4), and stored at 4°C.

(4) Clean-up with immobilized antibody column

A polypropylene column (Sepacolmini pp, Seikagaku Kogyo Co) was packed with 0.5 ml by gel volume of the immobilized antibody. The column was washed with water and acetonitrile, which was then replaced with PBS. The column was loaded in various experimental runs with 1 ml of a plasma test sample, standard 11-dhTXB$_2$ at various concentrations and solution of plasma test sample to which standard 11-dhTXB$_2$ had been added. After the column was washed with water (5 ml, x 3), elution was effected with 5 ml of acetonitrile and the eluate was evaporated to dryness to provide a test sample for enzyme immunoassay.

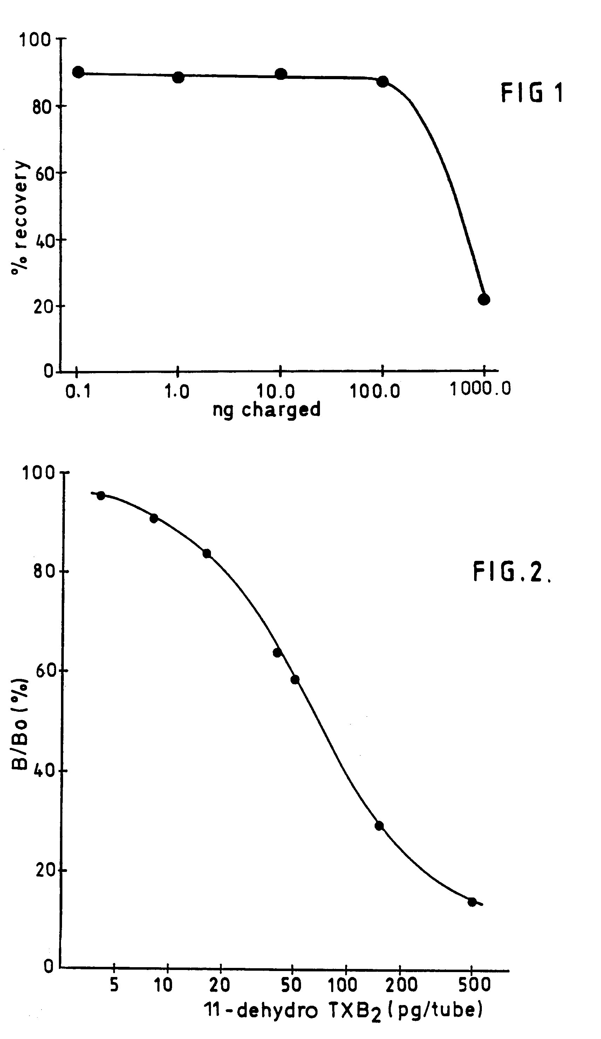

The recovery achieved by the use of this immobilized antibody column is shown in Figure 1, where the vertical axis shows the recovery (%) against the load shown by the horizontal axis. As shown in the Figure 1, 11-dhTXB$_2$ at a level of 0.1 to 100 ng can conveniently be pretreated at a good rate of recovery.

(5) Preparation of 11-dhTXB$_2$ labelled with horseradish peroxidase (HRP)

A solution of 1.44 mg of 11-dhTXB$_2$ N-succinimidyl ester in 0.4 ml of dioxane and a solution of 4 mg of HRP in 0.8 ml of PB were mixed with ice-cooling, and the resulting mixture was stirred at 4°C for 4 hours. To the mixture was added 2.8 ml of PB, and then the mixture was dialyzed against PB at 4°C for 48 hours. To the dialyzed solution was added PB containing 0.1% of gelatin and 0.9% of sodium chloride, in order to adjust the HRP concentration to 500 μg/ml. The labelled material was stored at 4°C.

(6) Assay of 11-dhTXB$_2$ in plasma

The test sample prepared in (4) was dissolved in 0.1 ml of 50mM Tris-buffer containing 0.1% gelatin and 0.9% sodium chloride (pH 9.2, hereinafter referred to as "Buffer A"). To the resulting solution were added 0.1 ml of the first antibody as in Example 1(7)(c) and 0.1 ml of the HRP-labelled 11-dhTXB$_2$ as in Example 1(7)(d), and reaction was carried out at 4°C for 4 hours. To the reaction mixture was then added 0.1 ml of the second antibody as in Example 1(7)(e) and, after mixing, the reaction was carried out at 4°C

EP 0 336 647 B1

for 16 hours. After completion of the reaction, 1.5 ml of PBS was added thereto and mixed, followed by centrifugation and removal of the supernatant by aspiration. To the centrifugate was again added 1.5 ml of PBS and the same operations were repeated. After removal of the supernatant, the assay for enzyme actvity was performed. 1.8 ml of 3,3′,5,5′-tetramethylbenzidine solution (0.01% in 50 mM acetate-citrate buffer containing 3% dimethyl sulfoxide (pH 5.5)) and 0.2 ml of aqueous hydrogen peroxide were added, and, after mixing, the reaction was carried out at 37°C for 30 minutes. The reaction was stopped by addition of 2 ml of 0.5 M sulfuric acid. Absorbance of the resultant solution at 450 nm was then measured.

In this way, the calibration curve shown in Figure 2 was obtained, where $B/B_0$ on the verticla axis is shown against the amount of 11-dhTXB$_2$ on the horizontal axis. The value of $B/B_0$ was calculated using the formula:

$$B/B_0 \ (\%) = 100 \ x \ (\text{enzyme activity with 11-dhTXB}_2)/(\text{enzyme activity without 11-dhTXB}_2).$$

(7) Make-up of kit for quantitation of 11-dhTXB$_2$ (sufficient for 100 assays)

A kit for 100 assays is made up of the following components:
(a) Buffer A: 100 ml;
(b) Standard 11-dhTXB$_2$: a solution of 0.1 mg of the lactone form of 11-dhTXB$_2$ in 1 ml of acetonitrile, dilution being effected with Buffer A;
(c) First Antibody: the antiserum obtained in Example 1(2), diluted 20,000-fold with Buffer A, 10 ml;
(d) HRP-labelled 11-dhTXB$_2$: the labelled material obtained in Example 1(5), diluted with buffer A containing 0.5% normal rabbit serum (Daiichi Radioisotope Kenkyusho) to 20 ng/ml, 10 ml;
(e) Second Antibody: goat anti-rabbit $\gamma$-globulin antiserum (Daiichi Radioisotope Kenkyusho) diluted 30-fold with Buffer A containing 0.3% EDTA, 10 ml;
(f) Enzyme Substrate: a solution of 0.01% 3,3′,5,5′-tetramethylbenzidine in 50 mM acetate-citrate buffer containing 3% dimethyl sulfoxide (pH 5.5), 180 ml, and 0.02% aqueous hydrogen peroxide, 20 ml;
(g) Enzyme Reaction Stopping Solution: 0.5M sulfuric acid, 200 ml;
(h) Buffer for washing immunoprecipitates: PBS, 300 ml;
(i) Column and immobilized first antibody; 100 disposable columns packed with packing material comprising immobilized first antibody (alternatively the 100 columns need not be prepacked and can be supplied with separate packing material, or a single re-usable column can be supplied in place of the 100 disposable columns.

With the use of such kits, for clean up of samples and enzyme immunoassay of 11-dhTXB$_2$, the assay of 11-dhTXB$_2$ within the plasma range of 5 to 500 pg/ml is possible. By the use of the selective clean-up pretreatment method, it has become possible efficiently to remove substances which exhibit cross-reactivity or interfere with the antigen-antibody reaction of the enzyme immunoassay.

Example 2

Preparation of immobilized monoclonal antibody for 11-dhTXB$_2$

(1) Immunization and cell fusion:

300 $\mu$g of 11-dhTXB$_2$ was reacted with 3 mg of bovine serum albumin (BSA) using the N-succinimidyl ester method, and the resultant product was administered intraperitoneally into BALB/C mice of 6 to 8 weeks of age in an amount of 100 $\mu$g (by weight of protein). Immunization was effected by injection 4 times every 2 weeks, and on the third day after the final immunization, the spleen of the mouse was taken out, the spleen lymphocytes and myeloma cells (SP2) were subjected to cell fusion by the polyethylene glycol method, and hybridomas were selected in HAT medium.

(2) Screening of antibody-producing cells:

11-dhTXB$_2$ esterified with $^3$H methyl iodide was prepared as the labelled antigen for screening of the ability to produce antibody. In order to separate the desired $^3$H-methyl-11-dhTXB$_2$ from $^3$H-methyl iodide and unreacted 11-dhTXB$_2$, reverse phase HPLC was employed with a $\mu$-Bondapack C$_{18}$ column and as solvent a mixture of acetonitrile/water/acetic acid (35:65:0.1, v/v/v) adjusted to pH 6.5 with aqueous ammonia.

11

Screening was performed by the use of a serum-free medium (Uitroser G, IBF), and antibody-producing ability was detected at a ratio of one in every fifteen wells.

(3) Cloning:

Cloning was performed by the soft agar method in which a very small number of the desired antibody-producing cells were seeded on agar medium.

(4) Collection of monoclonal antibody:

In conventional manner, the hybridoma was grown in a mouse abdomen, the ascites was collected, and the IgG fraction was obtained by use of ammonia sulfate fractionation and Protein A Sepharose (Trade Mark) chromatography.

The cross-reactivity (%) of the antibody was determined as shown in the following Table 2:

Table 2

| | |
|---|---|
| 11-dhTXB$_2$ (acid form) | 100 |
| TXB$_2$ | 0.03 |
| 15-keto-PGF$_{2\alpha}$ | 0.05 |
| 2,3-dinor-TXB$_2$ | <0.02 |
| PGB$_2$ | <0.02 |
| PGD$_2$ | <0.02 |
| PGE$_2$ | <0.02 |
| PGF$_{2\alpha}$ | <0.02 |
| 6-keto-PGF$_{1\alpha}$ | <0.02 |
| PGE$_2$-MPM | <0.02 |
| PGF$_{2\alpha}$-MPM | <0.02 |

(5) Preparation of immobilized monoclonal antibody column

5.7 g of Sepharose 4B activated by cyanogen bromide according to the method described in Example 1(3) was suspended in 20 ml of 0.1M carbonate buffer (pH 9.0), 100 mg of the IgG fraction of anti-11-dhTXB$_2$ monoclonal antibody was added, and the mixture was reacted at 4°C for 16 hours. The mixture was washed with 0.1M Tris-hydrochloride buffer (pH 8.0) which was then replaced with PBS (pH 7.4). The concentration of IgG of the immobilized antibody was adjusted to 375 $\mu$g per ml gel by adding 70 g of untreated Sepharose 4B and stored at 4°C. This immobilized antibody was packed in an amount of 0.8 ml by gel volume in a column made of polypropylene (Sepacol mini PP, Seikagaku Kogyo Co). The column was washed with water and acetonitrile, which was then replaced with PBS.

(6) Characteristics of the immobilized monoclonal antibody

The column was loaded with 1 ml of plasma test sample or standard 11-dhTXB$_2$ at various concentrations both spiked with a constant amount of $^3$H-labelled 11-dhTXB$_2$ (Amersham) as tracer. After the column was washed with PBS (10 ml) and water (5 ml), elution was achieved with 5 ml of methanol:water (95:5 v/v%). An aliquot of the eluate was measured using a liquid scintillation counter to estimate the recovery of the immobilized column.

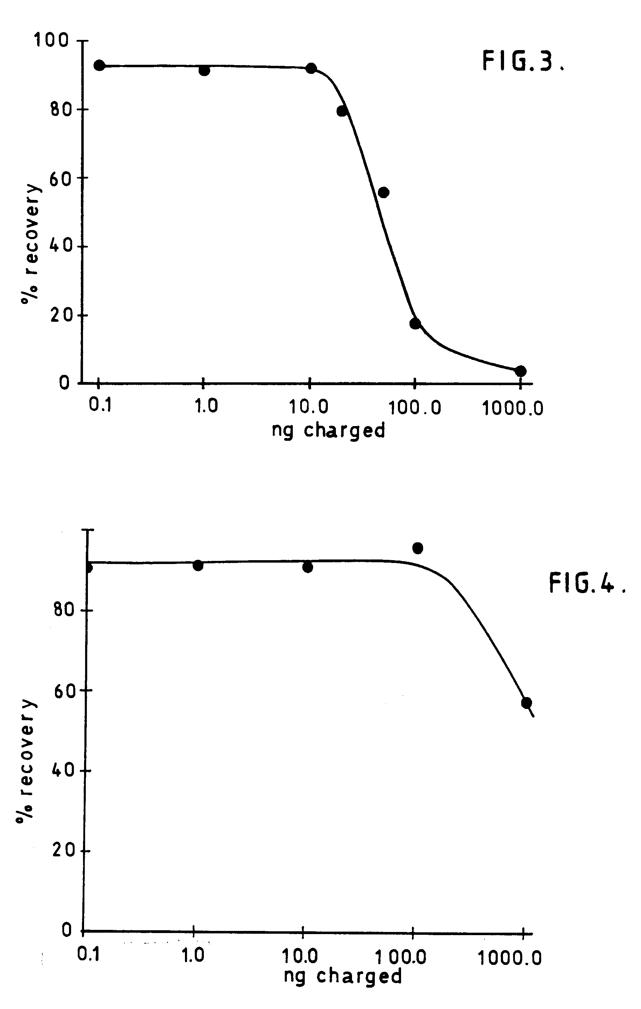

The recovery achieved by the use of this immobilized antibody column is shown in Figure 3, where the vertical axis shows the recovery (%) against the load shown by the horizontal axis.

Example 3

Assay of prostaglandin $E_2$ ($PGE_2$) in urine

(1) Preparation of $PGE_2$ antigen

15 mg of $PGE_2$ was esterified using the procedure of Example 1(1). The resultant saline antigen solution was stored at -20°C.

(2) Preparation of $PGE_2$ monoclonal antibody

The procedure of Example 2 was followed using a $PGE_2$-BSA conjugate as immunogen.
The cross-reactivity (%) of the antibody was determined as shown in the following Table 3:

Table 3

| | |
|---|---|
| $PGE_2$ | 100 |
| $PGE_1$ | 7.0 |
| $PGD_2$ | 1.0 |
| $PGA_2$ | 0.2 |
| $PGB_2$ | 1.0 |
| $PGF_{2\alpha}$ | 4.3 |
| $PGF_{1\alpha}$ | 0.1 |
| 6-keto-$PGF_{1\alpha}$ | 5.4 |
| 13,14-dihydro-15-keto-$PGE_2$ | <0.1 |
| 13,14-dihydro-15-keto-$PGF_{2\alpha}$ | <0.1 |
| $5\alpha,7\alpha$-dihydro-11-keto-tetranorprostane-1,16-dioic acid | <0.1 |
| 15-keto-$PGF_{2\alpha}$ | <0.1 |
| $TXB_2$ | <0.1 |

(3) Preparation of immobilized antibody column

The monoclonal antibody was dialyzed and immobilized by the procedure of Example 1(3).

(4) Clean-up with immobilized antibody column

The procedure of Example 1(4) was followed, except that the test volume was 1 ml urine, the gel volume was 1.0 ml, and the elution solvent was methanol.
The recovery achieved by the use of this immobilized antibody column is shown in Figure 4, where the vertical axis shows the recovery (%) against the load shown by the horizontal axis. As shown in the Figure 4, $PGE_2$ at a level of 0.1 to 100 ng can conveniently be pretreated at a good rate of recovery.

(5) Preparation of mimic antigen labelled with enzyme

According to the method described in Yamamoto et al. (Anal Biochem (1988) 168, 285), 9-deoxy-9-methylene-$PGF_{2\alpha}$ labelled with $\beta$-galactosidase was prepared.

(6) Assay of $PGE_2$ in urine

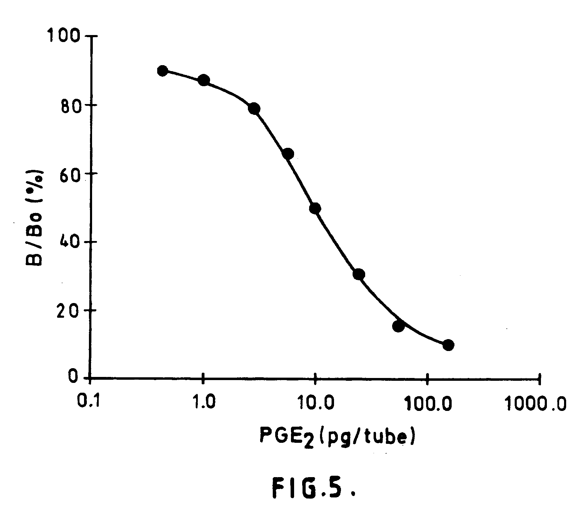

According to the method described in Yamamoto et al. (Anal Biochem (1988) 168, 287), $PGE_2$ in urine was determined. The calibration curve shown in Figure 5 was obtained:

$B/B_0$ (%) = 100 x (enzyme activity with $PGE_2$)/(enzyme activity without $PGE_2$).

13

EP 0 336 647 B1

(7) Make-up of kit for quantitation of $PGE_2$ (sufficient for 100 assays)

A kit for 100 assays contains the following components:

(a) Buffers:

(i) Buffer A, 10 mM phosphate buffer containing 0.1M sodium chloride, 1 mM magnesium chloride, 0.1% sodium azide and 0.1% ovalbumin (pH 7.0), 100 ml;

(ii) Buffer B, a buffer having the same composition as Buffer A except that 0.1% ovalbumin is omitted (pH 7.0), 10 ml;

(b) Standard Prostaglandin $E_2$: a solution of 0.1 mg of $PGE_2$ dissolved in 1 ml of ethanol, dilution being effected with Buffer B;

(c) First Antibody Immobilized Tube: tubes are prepared according to the method described by Yamamoto et al. (Anal Biochem (1988) 168, 285), in which a first antibody is adsorbed to a polystyrene tube, 100 tubes;

(d) Enzyme-Labelled Mimic Compound: 9-deoxy-9-methylene-$PGF_{2\alpha}$ labelled with $\beta$-galactosidase, a 20 ng/ml dilution of the antigen prepared in Example 4(5) diluted with the buffer A, 10 ml;

(e) Enzyme Substrate: Buffer A containing 0.1 mM 4-methylumbelliferyl-$\beta$-D-galactoside, 30 ml;

(f) Enzyme Reaction Stopping Solution: 0.1M glycine-sodium hydroxide buffer (pH 10.3), 250 ml;

(g) Column and immobilized first antibody, 100 disposable columns packed with packing material comprising immobilized first antibody (alternatively the 100 columns need not be prepacked and can be supplied with separate packing material, or a single re-usable column can be supplied in place of the 100 disposable columns.

With the use of such kits, for clean up of samples and enzyme immunoassay of $PGE_2$, the assay of $PGE_2$ within the urine range of 10 to 1000 pg/ml is possible. By the use of the selective clean-up pretreatment method, it has become possible efficiently to remove substances which exhibit cross-reactivity or interfere with the antigen-antibody reaction of the enzyme immunoassay.


Example 4

Assay of 11-$dhTXB_2$ by the double antibody solid phase (DASP) method

(1) Preparation of first antibody

Anti-11-$dhTXB_2$ antibody was obtained by the method described in Example 1(1) and 1(2).

(2) Preparation of enzyme-labelled antigen

11-$dhTXB_2$ labelled with horseradish peroxidase (HRP) was obtained by the method described in Example 1(4).

(3) Preparation of solid phase second antibody (DASP balls)

From a goat anti-rabbit $\gamma$-globulin antiserum (produced by Daiichi Radioisotope Co.), an IgG fraction was prepared by sodium sulfate fractionation and DEAE-cellulose column chromatography. 3000 polystyrene balls were immersed in a solution of 500 ml of 0.05 M phosphate buffer (pH 7.4) containing the IgG dissolved to a concentration of 1 mg/ml, and left to stand at room temperature for 2 hours, and further at 4°C overnight to effect adsorption, thus obtaining the desired DASP balls.

(4) Measurement of 11-$dhTXB_2$ by the DASP method

Kits for quantitation of 11-$dhTXB_2$ were prepared as described in Example 1(7), excepting that the DASP balls of Example 5(3) were employed as the component (e), Second Antibody. 11-$dhTXB_2$ was readily detected in the range from 5 to 500 pg/tube.

**Claims**

1. A method for determination of an analyte in a sample, the analyte being an arachidonic acid derivative which is selected from prostaglandins, thromboxanes and leukotrienes, the method comprising the steps of:

14

(a) providing an affinity chromatography column packed with a packing material comprising an immobilized first antibody, the first antibody being immunoreactive with the analyte;

(b) applying a sample to the column to adsorb to the packing material any analyte in the sample;

(c) washing the column to elute non-adsorbed sample;

(d) desorbing any adsorbed analyte from the column to give an assay fraction;

(e) assaying for analyte in the assay fraction using an enzyme immunoassay based on reagents including an assay antibody immunoreactive with the analyte, and an enzyme-labelled arachidonic acid derivative immunoreactive with the assay antibody.

2. A method according to claim 1, in which the reagents for the enzyme immunoassay further include a second antibody which is an antiglobulin immunoreactive with the assay antibody.

3. A method according to claim 2, in which the enzyme immunoassay is performed as a double antibody liquid phase assay.

4. A method according to claim 2, in which the enzyme immunoassay is performed as a double antibody solid phase assay.

5. A method according to claim 1, in which the enzyme immunoassay is performed as a solid phase antibody assay.

6. A method according to any preceding claim, wherein the first antibody is a polyclonal antibody.

7. A method according to any of claims 1 to 5, wherein the first antibody is a monoclonal antibody.

8. A method according to any preceding claim, wherein the sample is a body fluid sample.

9. A method according to claim 8, wherein the analyte is selected from $PGD_2$, $PGE_2$, $PGF_{2\alpha}$, $TXB_1$, $TXB_2$, $TXB_3$, $LTB_4$, $LTC_4$, $LTD_4$, and metabolites thereof.

10. A method according to claim 8, wherein the analyte is selected from $PGE_2$ or $11\text{-}dhTXB_2$.

11. A method according to any of claims 1 to 8, wherein the analyte is selected from prostaglandins and thromboxanes.

12. An enzyme immunoassay of a sample for an arachidonic acid derivative which is selected from prostaglandins, thromboxanes and leukotrienes, characterized by cleaning up the sample using affinity chromatography with immobilized antibody for the arachidonic acid derivative and using, in the cleaned sample, an assay antibody immunoreactive with arachidonic acid derivative and an enzyme-labelled arachidonic acid derivative immunoreactive with the assay antibody.

13. A kit for determination of an analyte in a sample, the analyte being an arachidonic acid derivative which is selected from prostaglandins, thromboxanes and leukotrienes, the kit including:

(a) an affinity chromatography column and a packing material comprising an immobilized first antibody, the first antibody being immunoreactive with the analyte;

(b) an assay antibody immunoreactive with the analyte; and

(c) an enzyme-labelled arachidonic acid derivative immunoreactive with the assay antibody.

14. A kit according to claim 13, which further includes

(d) a second antibody which is an antiglobulin immunoreactive with the assay antibody.

15. A kit according to claim 14, wherein the second antibody is not immobilized on a solid phase support, for a double antibody liquid phase assay.

16. A kit according to claim 14, wherein the second antibody is immobilized on a solid phase support, for a double antibody solid phase assay.

**Patentansprüche**

1. Verfahren zur Bestimmung eines Analyten in einer Probe, der ein unter Prostaglandinen, Thromboxanen und Leukotrienen ausgewähltes Arachidonsäurederivat ist, welches die Schritte umfaßt :

   (a) Bereitstellen einer Affinitätschromatographiesäule, die mit einem Füllmaterial gepackt ist, das einen mit dem Analyten immunoreaktiven, immobilisierten, ersten Antikörper enthält,

   (b) Auftragen einer Probe auf die Säule, wobei jeglicher in der Probe enthaltene Analyt an das Füllmaterial adsorbiert wird,

   (c) Waschen der Säule, wobei die nicht adsorbierte Probe eluiert wird,

   (d) Desorbieren jeglichen adsorbierten Analyten von der Säule, wobei eine Analysefraktion erhalten wird,

   (e) Untersuchen der Analysefraktion auf den Analyten unter Verwendung eines Immunoassays, der auf Reagentien basiert, welche einen mit dem Analyten immunoreaktiven Assay-Antikörper und ein mit einem Enzym markiertes Arachidonsäurederivat, das mit dem Assay-Antikörper immunoreaktiv ist, einschließen.

2. Verfahren nach Anspruch 1, bei dem die Reagentien für den Enzym-Immunoassay weiter einen zweiten Antikörper umfassen, der ein mit dem Assay-Antikörper immunoreaktives Antiglobulin ist.

3. Verfahren nach Anspruch 2, bei dem der Enzym-Immunoassay als Doppelt-Antikörper-Flüsssigphasen-Assay durchgeführt wird.

4. Verfahren nach Anspruch 2, bei dem der Enzym-Immunoassay als Doppelt-Antikörper-Festphasen-Assay durchgeführt wird.

5. Verfahren nach Anspruch 1, bei dem der Enzym-Immunoassay als Festphasen-Antikörper-Assay durchgeführt wird.

6. Verfahren nach einem der vorhergehenden Ansprüche, bei dem der erste Antikörper ein polyklonaler Antikörper ist.

7. Verfahren nach einem der Ansprüche 1 bis 5, bei dem der erste Antikörper ein monoklonaler Antikörper ist.

8. Verfahren nach einem der vorhergehenden Ansprüche, bei dem die Probe eine Körperflüssigkeit ist.

9. Verfahren nach Anspruch 8, bei dem der Analyt unter $PGD_2$, $PGE_2$, $PGF_{2\alpha}$, $TXB_1$, $TXB_2$, $TXB_3$, $LTB_4$, $LTC_4$, $LTD_4$ und Metaboliten davon ausgewählt wird.

10. Verfahren nach Anspruch 8, bei dem der Analyt unter $PGE_2$ oder 11-dh$TXB_2$ ausgewählt wird.

11. Verfahren nach einem der Ansprüche 1 bis 8, bei dem der Analyt unter Prostaglandinen und Thromboxanen ausgewählt wird.

12. Enzym-Immunoassay einer Probe auf ein unter Prostaglandinen, Thromboxanen und Leukotrienen ausgewähltes Arachidonsäurederivat, dadurch gekennzeichnet, daß die Probe unter Verwendung von Affinitätschromatographie mit einem gegen das Arachidonsäurederivat gerichteten, immobilisierten Antikörper gereinigt wird und in der gereinigten Probe ein mit einem Arachidonsäurederivat immunoreaktiver Assay-Antikörper und ein mit dem Assay-Antikörper immunoreaktives Enzym-markiertes Arachidonsäurederivat verwendet wird.

13. Ausrüstung zur Bestimmung eines Analyten in einer Probe, der ein unter Prostaglandinen, Thromboxanen und Leukotrienen ausgewähltes Arachidonsäurederivat ist, welche umfaßt :

    (a) eine Affinitätschromatographiesäule und ein Füllmaterial, das einen immobilisierten, mit dem Analyten immunoreaktiven, ersten Antikörper enthält,

    (b) einen mit dem Analyten immunoreaktiven Assay-Antikörper, und

    (c) ein mit einem Enzym markiertes, mit dem Assay-Antikörper immunoreaktives Arachidonsäurederivat.

**14.** Ausrüstung nach Anspruch 13, welche weiter
(d) einen zweiten Antikörper enthält, der ein mit dem Assay-Antikörper immunoreaktives Antiglobulin ist.

**15.** Ausrüstung nach Anspruch 14 für einen Doppelt-Antikörper-Festphasen-Assay, bei dem der zweite Antikörper nicht auf einem Festphasen-Träger immobilisiert ist.

**16.** Ausrüstung nach Anspruch 14 für einen Doppelt-Antikörper-Festphasen-Assay, bei dem der zweite Antikörper auf einem Festphasen-Träger immobilisiert ist.

**Revendications**

**1.** Procédé de détermination d'une substance à analyser dans un échantillon, la substance à analyser étant un dérivé d'acide arachidonique choisi parmi les prostaglandines, les thromboxanes et les leucotriènes, selon lequel :
a) on prépare une colonne de chromatographie d'affinité garnie avec un matériau de garnissage comprenant un premier anticorps immobilisé, le premier anticorps étant immunoréactif avec la substance à analyser ;
b) on applique un échantillon dans la colonne, afin d'adsorber dans le matériau de garnissage, une quelconque substance à analyser dans l'échantillon ;
c) on lave la colonne afin d'éluer l'échantillon non adsorbé ;
d) on désorbe une quelconque substance à analyser adsorbée, à partir de la colonne afin d'obtenir une fraction d'essai ;
e) on détecte la substance à analyser dans la fraction d'essai, en employant un essai immunoenzymatique basé sur des réactifs comprenant un anticorps d'essai immunoréactif avec la substance à analyser, et un dérivé d'acide arachidonique marqué avec une enzyme, immunoréactif avec l'anticorps d'essai.

**2.** Procédé selon la revendication 1, dans lequel les réactifs de l'essai immunoenzymatique, comprennent en outre un deuxième anticorps qui est une anti-globuline immunoréactive avec l'anticorps d'essai.

**3.** Procédé selon la revendication 2, dans lequel l'essai immunoenzymatique est effectué sous la forme d'un essai en phase liquide à deux anticorps.

**4.** Procédé selon la revendication 2, dans lequel l'essai immunoenzymatique est effectué sous la forme d'un essai en phase solide à deux anticorps.

**5.** Procédé selon la revendication 1, dans lequel l'essai immunoenzymatique est effectué sous la forme d'un essai à anticorps en phase solide.

**6.** Procédé selon l'une quelconque des revendications précédentes, dans lequel le premier anticorps est un anticorps polyclonal.

**7.** Procédé selon l'une quelconque des revendications 1 à 5, dans lequel le premier anticorps est un anticorps monoclonal.

**8.** Procédé selon l'une quelconque des revendications précédentes, dans lequel l'échantillon est un échantillon de fluide corporel.

**9.** Procédé selon la revendication 8, dans lequel la substance à analyser est choisie parmi $PGD_2$, $PGE_2$, $PGF_{2\alpha}$, $TXB_1$, $TXB_2$, $TXB_3$, $LTB_4$, $LTC_4$, $LTD_4$, et les métabolites de ceux-ci.

**10.** Procédé selon la revendication 8, dans lequel la substance à analyser est choisie parmi $PGE_2$ ou $11$-$dhTXB_2$.

**11.** Procédé selon l'une quelconque des revendications 1 à 8, dans lequel la substance à analyser est choisie parmi les prostaglandines et les thromboxanes.

**12.** Essai immunoenzymatique d'un échantillon, eu égard à un dérivé d'acide arachidonique choisi parmi les prostaglandines, les thromboxanes et les leucotriènes, caractérisé en ce qu'on épure l'échantillon par chromatographie d'affinité, à l'aide d'un anticorps immobilisé, eu égard au dérivé d'acide arachidonique, et on emploie, dans l'échantillon épuré, un anticorps d'essai immunoréactif avec le dérivé d'acide arachidonique et un dérivé d'acide arachidonique marqué avec une enzyme immunoréactif avec l'anticorps d'essai.

**13.** Nécessaire d'analyse d'une substance à analyser dans un échantillon, la substance à analyser étant un dérivé d'acide arachidonique, choisi parmi les prostaglandines, les thromboxanes et les leucotriènes, comprenant :
a) une colonne de chromatographie par affinité et un matériau de garnissage comprenant un premier anticorps immobilisé, le premier anticorps étant immunoréactif avec la substance à analyser ;
b) un anticorps d'essai immunoréactif avec la substance à analyser ; et
c) un dérivé d'acide arachidonique marqué avec une enzyme, immunoréactif avec l'anticorps d'essai.

**14.** Nécessaire selon la revendication 13, comprenant en outre :
(d) un deuxième anticorps qui est une immunoglobuline immunoréactive avec l'anticorps d'essai.

**15.** Nécessaire selon la revendication 14, dans lequel le deuxième anticorps n'est pas immobilisé sur un support en phase solide pour effectuer un essai à deux anticorps en phase liquide.

**16.** Nécessaire selon la revendication 14, dans lequel le deuxième anticorps est immobilisé sur un support en phase solide pour effectuer un essai en phase solide à deux anticorps.

FIG 1

FIG.2.

FIG.3.

FIG.4.

FIG.5.